(19) Europäisches Patentamt
European Patent Office
Office européen des brevets

(11) **EP 1 511 857 B1**

(12) **EUROPEAN PATENT SPECIFICATION**

(45) Date of publication and mention of the grant of the patent:
**12.12.2007 Bulletin 2007/50**

(51) Int Cl.:
*C12Q 1/68* (2006.01)     *A01K 67/027* (2006.01)
*C12N 15/11* (2006.01)

(21) Application number: **03724756.6**

(22) Date of filing: **30.05.2003**

(86) International application number:
**PCT/CA2003/000831**

(87) International publication number:
**WO 2003/102230 (11.12.2003 Gazette 2003/50)**

(54) **PTHRP-BASED PREDICTION AND DIAGNOSIS OF BONE DISEASE**

PTHRP-BASIERTE VORHERSAGE UND DIAGNOSE VON KNOCHENKRANKHEIT

PREDICTION ET DIAGNOSTIC DE MALADIES OSSEUSES BASEES SUR PTHRP

(84) Designated Contracting States:
**AT BE BG CH CY CZ DE DK EE ES FI FR GB GR HU IE IT LI LU MC NL PT RO SE SI SK TR**
Designated Extension States:
**LT**

(30) Priority: **31.05.2002 US 384122 P**

(43) Date of publication of application:
**09.03.2005 Bulletin 2005/10**

(73) Proprietor: **McGill University
Montreal,
Quebec H3A 2T5 (CA)**

(72) Inventors:
• **KARAPLIS, Andrew
Kirkland, Québec H9J 3V6 (CA)**
• **GOLTZMAN, David
Westmount, Québec H3Y 2W3 (CA)**

(74) Representative: **Beetz & Partner
Steinsdorfstrasse 10
80538 München (DE)**

(56) References cited:
WO-A-00/18954     WO-A-00/23594
WO-A-01/85784

• HE B ET AL: "Tissue-specific targeting of the Pthrp gene: The generation of mice with floxed alleles" ENDOCRINOLOGY, vol. 142, no. 5, 2001, pages 2070-77, XP002256104
• PAUSOVA Z ET AL: "Molecular characterization of an intragenic minisatellite (VNTR) polymorphism in the human parathyroid hormone-related peptide gene in chromosome region 12p12.1-p11.2" GENOMICS, vol. 17, 1993, pages 243-44, XP002256105
• LANSKE B ET AL: "ABLATION OF THE PTHrP GENE OR THE PTH/PTHrP RECEPTOR GENE LEADS TO DISTINCT ABNORMALITIES IN BONE DEVELOPMENT" JOURNAL OF CLINICAL INVESTIGATION, NEW YORK, NY, US, vol. 104, no. 4, August 1999 (1999-08), pages 399-407, XP002906912 ISSN: 0021-9738

**Description**

## TECHNICAL FIELD

The invention relates to methods and materials for the prediction, diagnosis and treatment of disease. More particularly, the present invention relates to methods and materials for the prediction, diagnosis and treatment of bone disease(s). The present invention also describes a system and method for screening for compounds having therapeutic potential in the treatment of bone disease(s), and in particular, osteoporosis.

## BACKGROUND OF THE INVENTION

Bone diseases affect women, men, and children of all ages. From infancy to old age, bone disease profoundly alters the quality of life for millions of North Americans. Each year, osteoporosis, Paget's disease, osteogenesis imperfecta and multiple myeloma, among other bone diseases, strike more than 30 million people in the USA alone and cause loss of independence, disability, pain, and death. The annual cost of acute and long-term care relating to bone diseases in the United States is estimated to be $20 billion. As the population ages, these costs are expected to increase to more than $60 to $80 billion by the year 2020. Without intervention, including improved methods of diagnosis, especially pre-onset prognostic tests and potential prophylactic treatments, chronic diseases, such as osteoporosis, will drive the cost of acute and long-term care well into the next century and overwhelm any effort to contain health care costs.

Osteoporosis and related fractures arising from diminished bone density are particularly common in older individuals and contribute substantially to the healthcare costs and burden of illness associated with the disease. Although osteoporosis has many causes, about 80% of the underlying etiology is genetic. Unfortunately, there are no tests commercially available currently that can determine an individual's predisposition for osteoporosis. Very often, an individual is diagnosed with osteoposoris only after the disease has progressed extensively. Failure to provide early detection of bone disease and/or a predisposition for bone disease drives up the cost and suffering associated with such a disease.

Recently, studies in the laboratories of the instant applicants and others, have provided compelling evidence that a protein expressed in the bone-forming cells of the skeleton (osteoblasts), named parathyroid hormone-related protein (PTHrP), is critical for the proper recruitment, proliferation, differentiation, and function of these cells, processes that are pivotal for maintaining proper bone density and preventing the development of osteoporosis(Horwitz et al. J Clin Endocrinol Meta. , February 2003,88 (2) 569-575).

The bulk of evidence generated by the applicants can be summarized as follows: Mice homozygous for PTHrP gene inactivation (Karaplis et al. , (1994) Genes Dev. 8, 277-289; Amizuka et al. , (1994) J. Cell Biol. 126,1611- 1623) were observed to possess dyschondroplastic skeletal abnormalities and altered endochondral bone formation, that culminate in their death in the immediate peripartum period. On the other hand, mice heterozygous for PTHrP gene inactivation (knockout mice missing one copy of the PTHrP gene from its genome when normally there are two) are phenotypically normal at birth, but develop, by 3 months of age, features consistent with premature osteoporosis (Amizuka et al. , (1996) Developmental Biol. 175,166-176). This severe form of osteoporosis is associated with decreased PTHrP expression in the skeleton and characterized by a marked decrease in trabecular bone volume and connectivity (decreased degree of anisotropy and increased structure model index), observations very characteristic of the human form of osteoporosis.

A method for diagnosing bone disease in a patient is proposed in international patent application publication WO00/18954 (Karaplis et al.) whereby the expression level of PTHrP in a biological sample is correlated with the predisposition for the disease.

As such, it has been demonstrated that the level of PTHrP correlates with osteoporotic bone disease. However, there remains a need for improved methods and systems for characterizing the basis of bone disease, to provide further insight into the mechanism of such diseases, and to develop sensitive diagnostic and treatment methods relating thereto. Furthermore, there remains a need for improved methods for detecting bone disease prior to the onset of the disease and/or providing means to determine a genetic predisposition thereto so as to allow for the implementation of corresponding genotype-specific customized treatment and/or prophylactic regimes to improve health of an affected individual.

## OBJECTS OF THE INVENTION

It is an objective of the present invention to provide a method for diagnosing bone disease in an individual.

It is a further objective of the present invention to provide a method for characterizing a predisposition for and/or susceptibility to bone disease in an individual.

It is a further objective of the present invention to provide a method for selectively treating bone disease and/or a predisposition for bone disease in an individual diagnosed therewith.

It is yet a further object of the present invention to provide a method for screening for novel therapeutics for the treatment of bone disease.

The above objects are achieved by the invention as defined in the independent claims.

## SUMMARY OF THE INVENTION

In accordance with an embodiment of the present invention indicators of bone disease have been characterized. More specifically, genetic indicators of bone disease have been characterized within the PTHrP gene. In particular, a genetic indicator of osteoporosis has been characterized within the PTHrP gene in accordance with a preferred embodiment of the present invention. Furthermore, the indicators of the present invention provide novel diagnostic and therapeutic targets for characterizing and/or treating a predisposition for bone disease, such as osteoporosis, for example. In addition, the indicators of bone disease, as identified in accordance with the present invention, provide novel targets for modulating expression of the PTHrP gene in connection with providing a treatment and/or prophylactic regime in an individual in need thereof. As described in accordance with one embodiment of the present invention, indicators of bone disease occur within a variable number of tandem repeat (VNTR) region within an intron of the PTHrP gene. Preferably, the intron is located between exons VI and VII of the PTHrP gene. APTHrP gene of the present invention preferably refers to a mammalian PTHrP gene. In accordance with one embodiment of the present invention, a PTHrP gene refers to a murine PTHrP gene. More preferably, a PTHrP gene of the present invention refers to a human PTHrP gene.

In accordance with an embodiment of the present invention, an indicator of the present invention is a genetic segment within a VNTR region of the PTHrP gene. Preferably, this genetic segment is characterized as an indicator of bone disease or an indicator of a predisposition for bone disease on the basis of allele length. More preferably, a genetic indicator of the present invention is characterized according to the number of predetermined repeat sequences within the VNTR region of the PTHrP gene. One embodiment of the present invention characterizes a genetic indicator of bone comprising at least one 9-mer nucleotide sequence within the VNTR region of the PTHrP gene. Preferably, a genetic indicator comprises at repeat of two or more 9-mer nucleotide sequences of the present invention.

The term "genetic indicator" is intended to mean a marker within a gene of interest that provides an indication of an individual's genetic predisposition and/or potential to develop a disease in the individual identified therewith. Preferably, a genetic indicator of the present invention refers to a marker within a PTHrP gene that provides an indication of an individual's genetic predisposition and/or potential to develop bone disease in the individual identified therewith.

The term "allele" is intended to mean an alternative form of a genetic segment or a region of a gene of interest that provides a genetic indicator in accordance with the present invention. Preferably, an allele of the present invention is a form of the variable number tandem repeat (VNTR) region of the PTHrP gene.

The term "diagnosis" or "diagnosing" refers to the identification of a disease state or a predisposition or susceptibility for developing a disease in a mammal, based on, at least in part, a genetic indication thereof. Accordingly, the term "diagnosis" or "diagnosing" may encompass a prognosis.

The term "bone disease state" refers to a degree of severity of the disease in an afflicted individual.

The term "individual" refers a mammal. Preferably, a mammal is a mouse. More preferably, a mammal is a human.

In accordance with one aspect of the present invention, there is provided a method of diagnosing bone disease and/or a predisposition therefore in an individual according to claim 1. A biological sample of the present invention is preferably includes DNA of said individual whereby the sample is suitable for amplifying and/or detecting the DNA contained therein.

One or more genetic indicators of the present invention may include a region within said PTHrP gene. According to one embodiment of the present invention, one or more genetic indicators of the present invention may comprise a variable number of tandem repeat (VNTR) region within said PTHrP gene. Furthermore, the one or more genetic indicators of the present invention may comprise of variable number of tandem repeat (VNTR) region (s) within said PTHrP gene wherein said region (s) is characterized on the basis of allele length. According to a preferred embodiment of the present invention, a genetic indicator is a segment of the variable number tandem repeat (VNTR) region of a PTHrP gene having a predetermined length wherein the genetic indicator correlates to a bone disease state and/or a susceptibility for bone disease. In accordance with an embodiment of the present invention, a bone disease is preferably osteoporosis.

The region of interest in a PTHrP gene of the present invention is the VNTR region. More preferably, this region includes a variable number of tandem repeats in the nucleotide sequence thereof. Furthermore, in accordance with embodiments of the present invention, a genetic indicator is preferably a segment of the PTHrP gene within the VNTR region that are between 252 to 460 base pairs in length.

According to a further preferred embodiment of the present invention, a genetic indicator includes a segment within the VNTR region of a PTHrP gene that is 252 base pairs (bp), 288bp, 332bp, 356bp, 378bp, 393bp, 414bp or 460bp in length. A genetic indicator of the present invention may also comprise at least one nucleotide repeat of a 9-mer nucleotide sequence within the PTHrP gene. More preferably, the 9-mer nucleotide sequence of the present invention may be further referred to herein as a variable number tandem repeat (VNTR) selected from the group consisting of GTATATATA and ATATATATA.

In accordance with one aspect of the present invention, there is provided a method of diagnosing a susceptibility for bone disease in an individual according to claim 13.

It is provided a method of treating a patient identified with bone disease or a susceptibility therefore, said patient having a PTHrP gene; said method comprising: (a) characterizing the PTHrP gene of said patient with respect to variations detected within a variable number tandem repeat (VNTR) region of said gene; and, (b) selectively treating the patient with a treatment regime corresponding to a genotypic profile of said patient; wherein said genotypic profile is characterized with respect to the detected variations within the VNTR region of said gene.

In accordance with still a further aspect of the present invention there is provided an isolated nucleic acid having a nucleotide sequence comprising one or more repeats of GTATATATA or ATATATATA wherein said nucleotide sequence has complimentarity to a region of a PTHrP gene. An isolated nucleic acid of the present invention may be used as a probe for a genetic indicator of bone disease.

Alternatively, an isolated nucleic acid of the present invention may be employed as a therapeutic agent in connection with a treatment or prophylactic regime provided to target bone disease.

In accordance with yet another aspect of the present invention, there is provided a commercial package for use in providing a diagnosis of bone disease and/or a risk of developing bone disease in an individual according to claim 20.

In embodiments, a bone disease of the present invention is a metabolic bone disease. More specifically, a bone disease of the present invention includes, but is not limited to osteoporosis, osteomalacia, osteopetrosis, Paget's disease and renal osteodystrophy.

In an embodiment, the characterization of a genetic indicator of the present invention comprises amplification of the VNTR region of aPTHrP gene, by PCR, for example, in accordance with methods well known in the art.

The invention further provides a use of a genetic indicator of the present invention to (a) diagnose bone disease in an individual; (b) determine if an individual has a predisposition to develop bone disease; or (c) both (a) and (b).

Further, a method of treating an individual having an PTHrP-gene is provided, comprising screening the individual for a genetic indicator of bone disease; characterizing a genotypic profile of the individual based on identification of one or more genetic indicators in the PTHrP gene; and, selectively treating the individual for bone disease if the genotypic profile of the individual is indicative of a risk of developing bone disease. According to an embodiment, selectively treating an individual may comprise developing a genotype- specific treatment regime for the individual that is tailored to the genotypic profile of that individual. A genotypic profile of the present invention is preferably a PTHrP- specific genotypic profile.

In accordance with another aspect, there is provided a transgenic non-human mammal homozygous for a missing PTHrP gene in osteoblast cells but not in non-osteoblast cells. Preferably, the transgenic non-human mammal is suitable for studying the development, treatment and prophylaxis of bone disease. Such a transgenic non-human mammal may have further application in the screening of compounds and/or agents having therapeutic and/or prophylactic effect on bone disease(s).

In accordance with another aspect, there is provided a single-stranded nucleic acid having a nucleotide sequence comprising one or more repeats of GTATATATA or ATATATATA wherein said nucleotide sequence has complimentarity to a region of a PTHrP gene.

In accordance with another aspect, there is provided a single-stranded oligonucleotide comprising of at least GTATATATA or ATATATATA for use in identifying candidate compounds having the ability to modulate PTHrP expression.

In accordance with another aspect, there is provided a single-stranded oligonucleotide comprising two or more repeats having a sequence of GTATATATA or ATATATATA for use in identifying candidate compounds having the ability to modulate PTHrP expression.

In accordance with another aspect of the present invention, there is provided a single-stranded oligonucleotide comprising the sequence of SEQ ID No: 1, or the complement thereof, for use in identifying candidate compounds having the ability to modulate PTHrP expression.

In accordance with another aspect of the present invention, there is provided an isolated nucleic acid comprising a sequence that hybridizes under stringent conditions to a hybridization probe the nucleotide sequence of which consists of SEQ ID No: 1 or the complement of SEQ ID No: 1.

In an embodiment, the somatic cells of the non- human mammal comprise osteoblast-specific PTHrP disruption means, such as a Cre-LoxP technology as described further herein below, capable of disrupting a PTHrP gene or a portion thereof specifically in osteoblast cells without disrupting the PTHrP gene in non-osteoblast cells. In an embodiment, such means comprise: (a) a Cre recombinase gene under control of an osteoblast-specific promoter (e.g. the type 1 collagen promoter); and (b) loxP sites which flank the PTHrP gene or a portion thereof (e.g. exon 4) (PTHrP flox/flox) (He et al. Endocrinology).

Furthermore, a transgenic non-human mammal is provided that is genetically engineered to be heterozygous for a missing PTHrP gene in osteoblast cells but not in non-osteoblast cells. Such an animal is generated in a manner similar to that disclosed for the homozygous osteoblast-specific knock-out mouse. The heterozygous osteoblast-specific knock-out mouse also provides a useful model for the study of the development, treatment and prophylaxis of bone disease. In particular, a heterozygous osteoblast-specific knock mouse is favourably suited for the screening of compounds and/or agents having therapeutic and/or prophylactic effect on bone disease(s). For example, in a heterozygous osteoblast-specific mouse model, osteoblast-specific compounds could be screened for their ability to modulate expression of the existing PTHrP gene.

## BRIEF DESCRIPTION OF THE DRAWINGS

Further features and advantages of the present invention will become apparent from the following detailed description, taken in combination with the appended drawings, in which:

Figure 1: Results of microCT analysis of bone from normal (left specimen) and heterozygous PTHrP (+/-) mice (right specimen), showing the diminished content of trabecular bone in the mutant animals.

Figure 2: Measured bone volume over total volume (BV/TV) in normal and heterozygous PTHrP (+/-) mice. B. V. Bone Volume, Tb.N, number of bone trabecules; Tb.Th., thickness of bone trabecules.

Figure 3: Results of analysis, in wild-type and heterozygous PTHrP (+/-) mice, of spacing between trabecules (Tb.Sp.),degree of anisotropy (DA), total volume (TV) and structure model index (SMI), features that describe the 3-D architecture of the bone.

Figure 4: Analysis of bone from wild-type and heterozygous PTHrP (+/-) mice via calcein staining followed by tetracycline (14 days later) staining. The distance between the two lines (Panels C and D) can be used to calculate the rate of bone formation, which is markedly diminished in the mutant mice.

Figure 5: Analysis of differetiation into osteoblasts. Bone marrow from wild-type (left) and heterozygous PTHrP (+/-) right) animals was extracted, cultured and induced to differentiate into osteoblasts (bone-forming cells), and subsequently stained with alizarin red which binds to calcified matrix.

Figure 6: Schematic representation of preparation of mice in which PTHrP is specifically inactivated in osteoblasts, according to an embodiment of the invention.

Figure 7: Analysis of bone from control mice (PTHrPflox/floxPTHrP++) and mutant mice lacking PTHrP in osteoblasts (PTHrP flo/flox/ostColl-).

Figure 8: Analysis of osteoblasts from control mice (left panel) and mutant mice lacking PTHrP in osteoblasts (right panel) via staining of apoptotic nuclei.

Figure 9: Structure of PTHrP genes from different species.

Figure 10: 252bp allele of PTHrP VNTR region. Regions corresponding to oligonucleotide primers used for PCR amplification are underlined. Tandem repeats (G/A TATATATA) are shown in bold.

Figure 11: Prevalence of PTHrP alleles in the general population. It is indicated on the figure that 16 of 19 osteoporotic male subjects tested (i.e. 84% of subjects) possessed the 252bp allele.

## DETAILED DESCRIPTION OF THE PREFERRED EMBODIMENTS

Osteoporosis is a very prevalent disorder with substantial morbidity and mortality affecting the aging population, as are many other bone diseases. A simple test that predicts early the potential risk for an individual to develop a bone disease, such as osteoporosis for example is very much needed and would be greatly served in accordance with embodiments of the present invention. As discussed further herein below, it has been identified in accordance with the present invention that the length of a variable number tandem repeat (VNTR) region in the PTHrP gene serves as a novel indicator of bone disease. Furthermore, a study conducted in accordance with the present invention examines the predictive value of the length of a VNTR region in the PTHrP gene on bone mineral density (BMD) in four groups of patients with normal and decreased BMDs: (1) osteoporotic males, and (2) osteoporotic premenopausal women, with both groups having a genetic etiology as the cause of osteoporosis, (3) healthy males, (4) healthy premenopausal women showing no signs of osteoporosis. The hypothesis is that the various alleles of the VNTR region of the PTHrP gene give rise to variable expression of the PTHrP protein within the bone microenvironment. Specifically, decreased expression of the PTHrP protein is herein correlated with decreased bone formation, and therefore diminished BMD, as discussed further herein below. Based on a characterization of an individual's genotypic profile with respect to bone disease, as provided in accordance with the identification of genetic indicators of the present invention, a corresponding genotype-specific treatment regime can be prescribed that is tailored to an individual's genotype.

Intermittent administration of Parathyroid Hormone Related Peptide (PTHrP) in vivo has an anabolic effect on bone formation (Stewart et al. J. Bone Miner Res 15:1517, 2000). The precise molecular and cellular basis for the anabolic actions of PTHrP are unclear, and efforts to elucidate these mechanisms in PTHrP knockout mice have been compromised due to the perinatal lethality of these animals prior to the present invention. To overcome this obstacle, described herein is a novel system enabling the selective inactivation of PTHrP in osteoblasts. Specifically, Cremediated recombination is used to selectively disrupt PTHrP expression in mouse osteoblasts.

This has been substantiated by several modalities in accordance with the present invention including classic histomorphometry and bone densitometry (DEXA was used in patients) as well as by more advanced techniques, including specifically computerized microtomography (mCT)of bone. The studies described herein utilize transgenic mice that have been generated in which the PTHrP gene has been selectively removed from osteoblasts but not other cells in the animal. These mice are shown to develop premature, severe osteoporosis. It is concluded in accordance with the present invention that the level of PTHrP expression within the bone microenvironment, specifically the osteoblasts, is critical for preventing osteoporosis. Furthermore, trangenic non-human mammals of the present invention further provide a novel system for studying bone disease that is very phenotypical of the actual human disease condition. Transgenic non-human mammals of the present invention also serve as novel models for studying the effects of candidate treatment regimes, such as compounds and/or agents for their effectiveness in treating, preventing or amelorating the progression of bone disease. In accordance with a preferred embodiment of the present invention, a system for evaluating and/or studying candidate therapeutics that modulate the expression of PTHrP in mammalian cells is provided. Osteoblast-specific PTHrP knock-out mammals of the present invention may be homozygous or heterozygous for a missing PTHrP gene in osteoblast cells.

The studies described herein of human PTHrP with respect to bone disease demonstrate that a specific region in the human PTHrP gene, herein referred to as a Variable Number of Tandem Repeats (VNTR) region, has a role in the regulation of PTHrP expression. In accordance with the present invention, the VNTR region of the PTHrP gene is identified as a novel marker for bone disease as herein described. In particular, a genetic indicator of bone disease and/or susceptibility for bone disease of the present invention may comprise the VNTR region of the PTHrP gene or a portion thereof.

While the existence of the VNTRs has been described, no role or function has been ascribed to it prior to the studies described herein. In the general population, this region varies in length from 252 to 460 nucleotides amongst individuals. In accordance with the present invention, it is observed that an allele having a longer VNTR region expresses PTHrP more efficiently than an allele having a shorter VNTR region. The VNTR region may therefore serve as a regulatory region for PTHrP gene transcription, as transcription factors and or other proteins may bind to this region.

According to one embodiment of the present invention, novel oligonucleotides are herein provided. According to a preferred embodiment , an oligonucleotide of the present invention is an osteoblast-specific oligonucleotide. Preferably, an oligonucleotide of the present invention includes at least a 9-mer nucleotide sequence as disclosed herein. More preferably, an oligonucleotide of the present invention includes a repeat comprising at least two 9-mer nucleotide sequences as disclosed herein. Oligonucleotides of the present invention are useful in characterizing novel therapeutic targets. An oligonucleotide of the present invention may be generated in accordance with well-known methodologies known in the art. Furthermore, an oligonucleotide of the present invention may be employed as a hybridization probe in accordance with embodiments of the present invention, and according to well known methods for achieving hybridization, such as those exemplified herein below.

In an analysis of a number of male subjects diagnosed with a genetic basis for osteoporosis, it was identified, in accordance with the present invention that 16 out of the 19 patients possessed a high risk allele, containing a shorter VNTR-containing region in the PTHrP gene, and likely less PTHrP expression within their skeletal microenvironment, consistent with the animal findings that lower PTHrP levels in osteoblasts is associated with the propensity to develop premature and severe forms of osteoporosis. Accordingly, in accordance with one embodiment of the present invention, a genetic indicator of bone disease and/or a predisposition for bone disease may be an allele of the PTHrP gene containing a VNTR region that is shorter than that of the average population. More preferably, a proportional relationship between VNTR length and predisposition to bone disease or severity of bone disease is identified in accordance with embodiments of the present invention. For example, the longer a VNTR region within a PTHrP gene of an individual of interest the less likely that individual is to develop a bone disease, while the shorter a VNTR region, the more likely an individual is to develop the disease. Alternatively, a diagnosis on the severity of an individual's bone disease may be provided in accordance with an embodiment of the present invention whereby when an individual identified as having bone disease or symptoms thereof is characterized as having a shorter allele of the VNTR region of the PTHrP gene, the individual may be diagnosed with a more severe form of bone disease than an individual identified as having a longer allele. Furthermore, an individual may be selectively treated for bone disease or a predisposition for bone disease in accordance with a genotypic characterization of the present invention. Preferably, a personal treatment regime corresponding with the individual's genotype is determined and prescribed.

A genetic test specific for characterizing alleles of the PTHrP gene, to identify individuals at risk for developing bone disease and in particular a genetic test to identify a predisposition for osteoporosis has not been described prior to the studies described herein.

Specifically, described herein is a correlation between certain alleles of the PTHrP gene, also herein referred to as genetic indicators, and bone disease. In accordance with the present invention alleles of the PTHrP gene have been identified as genetic indicators of bone disease and/or genetic indicators of a predisposition for bone disease. The alleles of the present invention are defined in part based on the size of a genomic region of the PTHrP gene, herein referred to as a VNTR containing region, and further in part based on the number of VNTRs contained within this region. A high risk allele, which is indicative of a greater risk of metabolic bone disease, comprises a shorter VNTR-containing region containing fewer VNTRs. In embodiments, the bone disease is more preferably a metabolic bone disease. According to a preferred embodiment of the present invention a metabolic bone disease may be selected from the group consisting of osteoporosis, osteomalacia, osteopetrosis,Paget's disease, and renal osteodystrophy.

The sequence of each VNTR may comprise:

RTATATATA

The above indicates that the first position of each VNTR may be a G or A, i.e. a VNTR may comprise:

GTATATATA or ATATATATA

Accordingly, nucleic acid sequences and/or oligonucleotides comprising one or more repeats of the nucleotide sequences outlined above are provided in accordance with the present invention. Isolated nucleic acids and/or oligonucleotides of the present invention may be employed as detection means, for genetic indicators of bone disease, such as hybridization probes for example. Furthermore, these aspects of the present invention serve as useful probes for identifying compounds with therapeutic or prophylactic potential in treating bone disease or a predisposition thereto. Alternatively, nucleic acid sequencces and/oroligonucleotides of the present invention may be administered in vivo as a preferred therapeutic and/or prophylactic treatment regime or component thereof for combating bone disease. The nucleic acid sequences and oligonucleotides of the present invention are prepared or isolated in accordance with methodologies well known in the art, for example some of such methodologies are described in the teachings of Sambrook et al. (1989, Molecular Cloning - A Laboratory Manual, Cold Spring Harbour Laboratories); Ausubel et al. (1994, Current Protocols in Molecular Biology, Wiley, New York); and Glick et al. (1994 Molecular Biotechnology - Principles and Applications of Recombinant DNA, ASM, Washington, D.C.). When employed as hybridization probes, the nucleic acid sequences and oligonucleotides of the present invention preferably hybridize with nucleic acids under stringent hybridization conditions, as well known in the art or as exemplified hereinbelow.

A correlation of between alleles of the VNTR region of the PTHrP gene with the level of PTHrP expression have been identified in accordance with the present invention. For example, the present invention has shown that alleles comprising longer VNTR- containing regions within the PTHrP gene correlate with higher levels of PTHrP. Furthermore, an increase in the number of repeats within the VNTR-region of the PTHrP gene have been correlated with higher levels of PTHrP expression, and thus, better bone formation. Accordingly, one aspect the invention provides for a genotyping assay for identifying a genetic indicator(i.e. a high risk allele) that is associated with low levels of PTHrP expression, to provide an indication that an individual having the genetic indicator is at risk for developing bone disease. In addition, the invention provides assays for identifying a genetic indicator associated with high levels of PTHrP (i.e. a low risk allele), to provide an indication that an individual having such an indicator is less likely to develop bone disease. For example, a 252bp allele of the VNTR containing region of a PTHrP gene is shown in accordance with the present invention to have a positive correlation with bone disease.

In accordance with this aspect of the present invention, a genetic indicator is characterized on the basis of length to have a genotypic basis for contributing to a disease or non-disease state in an individual whose genetic composition is determined to contain that indicator.

According to one embodiment, a degree of severity of a disease state may be characterized in accordance with a genetic indicator of the present invention.

The invention may be utilized to diagnose and/or treat individuals identified as being at risk of developing bone disease. For example, such individuals may exhibit one or more symptoms of bone disease or related conditions, or possess one or more of a group of risk factors associated with bone disease and/or related conditions. Individuals may for example be identified as at risk of bone disease on the basis of epidemiological criteria such as sex, age, socioeconomic factors or family history, on the basis of which an assessment may be made that the individual of interest is more likely than other persons to suffer from bone disease. Physicians typically diagnose bone disease based on the overall pattern of symptoms, medical history, family history, medications, physical exam, imaging methods (measurement of BMD, Bone Mineral Density) and a variety of blood and urine tests for determining bone integrity.

Thus, aspects of the present invention are methods for the diagnosis and prediction of metabolic bone disease, via assessing a PTHrP gene for predetermined genetic indicators appearing therein (the nature of a PTHrP allele present in a subject). Upon identification of a genetic indicator of the present invention, an individual's genotypic profile pertaining to bone disease can be characterized. A subject of the present invention is preferably a mammal, and more preferably a human. A subject of the present invention may be a subject at risk for metabolic bone disease.

In an embodiment, a genetic indicator is characterized based on the nature of the PTHrP VNTR containing region present in the allele of the PTHrP gene.

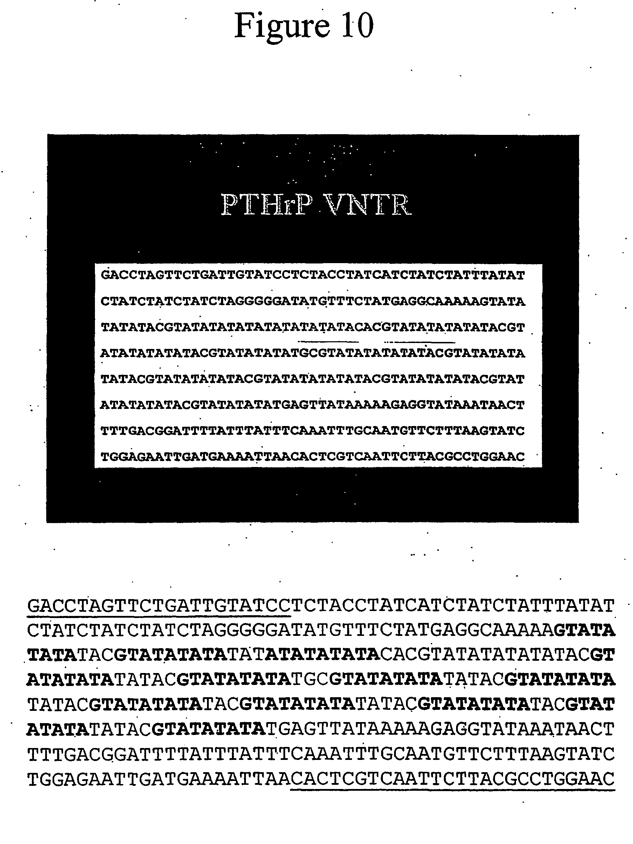

Accordingly, the invention provides a method of diagnosis or prediction of bone disease, the method comprising: a) obtaining a biological sample from said individual, said sample suitable for detecting a parathyroid hormone related peptide (PTHrP) gene of said patient therein; b) screening for one or more genetic indicators of bone disease in said PTHrP gene of said individual; and c) diagnosing said individual with respect risk for bone disease based on the results obtained in step (b). Thus, characterization of a genetic indicator identified in accordance with the present invention shall allow diagnosis of a bone disease or indicate that the patient has a predisposition for bone disease. According to a preferred embodiment of the present invention, a genetic indicator of the present invention is characterized on the basis of length. More preferably, a genetic indicator of the present invention includes a VNTR containing region of the PTHrP gene. Variants of the VNTR contaiing region of the PTHrP gene are also herein referred to as alleles or allelic variants, in accordance with embodiments of the present invention. In an embodiment, the genetic indicator occurs within a variable number of tandem repeat (VNTR)-region within an intron of the PTHrP gene. In an embodiment the intron is between exons VI and VII of the PTHrP gene. The characterization of a genetic indicator, in an embodiment of the present invention; may be based on the number of VNTRs present in an allele of PTHrP gene (e.g. 3 9-mer nucleotide sequence repeats). The characterization of a genetic indicator may in a further embodiment of the present invention be based on the identification of an allele selected from the group consisting of a 252bp, 288bp, 332bp, 356bp, 378bp, 393bp, 414bp and 460bp allele comprising a variable number of tandem repeat (VNTR)-region of the PTHrP gene.A PTHrP allele (or a genetic indicator based thereon) may be identified by various methods known in the art. For example, an allele comprising a VNTR region may be identified by amplifying this region via polymerase chain reaction (PCR) to obtain a PCR product, and examining the length of the PCR product obtained. Suitable oligonucleotide primers may be used for such amplification, for example those shown in Figure 10, as obtained from SHELDON BIOTECH, Montreal, PQ.

An allele may be identified for example by direct sequencing of the PTHrP gene or a region thereof, such as the VNTR containing region. Another method for identifying the PTHrP may be restriction fragment length polymorphism analysis (RFLP), for example by amplifying a nucleic acid sequence comprising the PTHrP gene or a region thereof, such as a VNTR containing region, by polymerase chain reaction (PCR) to obtain a PCR product, digesting the PCR product with a restriction enzyme to obtain a restriction digest product; and examining the length of the restriction digest product(s) produced, such as by gel electrophoresis, for example. Other methods may utilize hybridization of allele-specific probes under hybridization conditions which are optimized so that allelic differences may be detected. Microarray based methods may also be used, such as for example as described in US Patents 5,858,659 (Sapolsky et al.; January 12, 1999) or 6,223,127 (Berno; April 24, 2001).

In embodiments, a PTHrP allele is identified in a biological sample obtained from a subject, such as a tissue or body fluid of said subject. Suitable tissue or body fluids include but are not limited to blood, plasma, lymphocytes, epithelial cells, osteoblasts, bone marrow stromal cells, and fibroblasts. A biological sample of the present invention comprises DNA. Preferably, an individual's DNA can be detected and/or amplified from a biological sample of the present invention. DNA amplification and detection are carried out in accordance with methodologies well-known in the art, some of which are exemplified in the references.

In an embodiment, a nucleic acid comprising a PTHrP gene or portion thereof is purified from the sample prior to identifying the PTHrP allele therein. In an embodiment, the purified nucleic acid is amplified prior to determining the PTHrP allele present. In an embodiment, the purified nucleic acid is amplified with primers which amplify a PTHrP gene or a fragment thereof. In certain embodiments, such amplification is performed via PCR using primers which are designed such that they hybridize to sequences on either side of (i.e. 5' to and 3' to) the VNTR-containing region, for example, the primers shown in Figure 10.

The invention further relates to a use of an allele of a PTHrP gene, as described above, for the diagnosis and/or prediction of bone disease. In accordance with a preferred embodiment, the present invention provides a hybridization probe having sequence homology to at least a 9-mer nucleotide sequence of an allele of a PTHrP gene.

The invention further relates to commercial packages or kits for carrying out the diagnostic and predictive methods noted above, comprising the appropriate above-mentioned reagents (i.e. primers or probes, for example) together with instructions for methods for using such a commercial package for the purpose of diagnosising and/or predicting susceptibility for bone disease.

Accordingly, the invention further provides a commercial package or kit for use in the diagnosis and/or the prediction of bone disease. The commercial package or kit of the present invention may comprise means for detecting a genetic indictor of bone disease in a PTHrP gene of a subject of interest, together with instructions for characterizing said genetic indictor as a positive or negative indicator of bone disease and/or susceptability thereto. More preferably, the package or kit of the present invention includes quantitative correlation information relating predetermined genetic indicators to levels of disease severity and/or degrees of susceptibility thereto.

In one aspect, there is provided a method of treating a patient identified with bone disease or a susceptibility therefore, said patient having the PTHrP gene; said method comprising: (a) characterizing the PTHrP gene of said patient with respect to variations detected within a variable number tandem repeat (VNTR) region of said gene; and, (b) selectively treating the patient with a customized treatment regime corresponding to the detected variations within the VNTR region of said gene.

A variation in the PTHrP gene may be in the length of the VNTR region within the intron between exons VI and VII. Thus, such a variation would serve as a suitable genetic indicator in accordance with the present invention. The presence of a high risk allele (or genetic indicator) of the present invention may for example be taken as indicative of susceptibility or a predisposition to bone disease or to a more severe form of bone disease. More preferably, the present invention provides a method for selectively treating bone disease and/or a predisposition thereto according to an individual's genotype. That is, a genotype specific treatment or prophylactic regime to combat bone disease may be provided in accordance with the present invention. According to this embodiment of the present invention, a characterization of an individual's genotype is made according to genetic indicators of the present invention and a corresponding treatment or prophylactic regime is devised based thereon. For example, an individual identified to have a 252bp allele of the PTHrP gene, will be prescribed a corresponding treatment regime that is different from that prescribed to another individual identified to have 460 bp allele of the PTHrP gene, according to an embodiment of the present invention.

In accordance with various aspects of the invention, a patient may be treated for bone disease. For example, treating a patient for bone disease may comprise administering to the patient an effective amount of a compound or medicament. An effective amount of a medicament may be a therapeutically effective amount or a prophylactically effective amount. A "therapeutically effective amount" refers to an amount effective, at dosages and for periods of time necessary, to achieve the desired therapeutic result, such as reducing signs and symptoms of bone disease and/or delaying structural damage of bone. A therapeutically effective amount of a therapeutic may vary according to factors such as the disease state, age, sex, and weight of the individual, and the ability of the therapeutic to elicit a desired response in the individual. Dosage regimens may be adjusted to provide the optimum therapeutic response. A therapeutically effective amount is also typically one in which any toxic or detrimental effects of the therapeutic are outweighed by the therapeutically beneficial effects. A "prophylactically effective amount" refers to an amount effective, at dosages and for periods of time necessary, to achieve the desired prophylactic result, such as reducing signs and symptoms of bone disease and delaying structural damage of bone. A prophylactic dose may be used in subjects prior to or at an earlier stage of disease, and a prophylactically effective amount may be more or less than a therapeutically effective amount in some cases.

Medicaments for treating bone disease may for example include drugs approved by the FDA for treating patients with varying degrees of bone disease, such as drugs that reduce signs and symptoms of bone disease and delay structural damage of bone disease in patients. Such drugs may for example include: estrogen, alendronate, residronate, calcitonin, and parathyroid hormone.

In one aspect, the invention relates to a use in gene therapy of an PTHrP nucleic acid. The PTHrP nucleic acid may be delivered by a therapeutically acceptable gene therapy vector to modify a patient's PTHrP allele profile. Gene therapy may for example be used to replace a high risk PTHrP allele with a low risk PTHrP allele or enhance the expression of the PTHrP protein.

Gene therapy vectors may for example be an adeno-associated vector (AAV). Such a vector may comprise for example: a 5' inverted terminal repeat (ITR); a promoter, such as a CMV enhancer-promoter with a osteoblast-specific enhancer; an intron; a 3'-untranslated region (3'-UTR); a polyadenylation signal, such as an SV40 polyadenylation signal; and a 3'-ITR. For gene therapy vectors, the dosage to be administered may depend to a large extent on the condition and size of the subject being treated as well as the therapeutic formulation, frequency of treatment and the route of administration. Regimens for continuing therapy, including dose, formulation, and frequency may be guided by the initial response and clinical judgment. The parenteral route of injection into the interstitial space of tissue may be preferred, although other parenteral routes, such as inhalation of an aerosol formulation, may be required in specific administration. In some protocols, a formulation comprising the gene and gene delivery system in an aqueous carrier is injected 30 into tissue in appropriate amounts. The tissue target may be specific, for example the muscle or liver tissue, or it may be a combination of several tissues, for example the muscle and liver tissues. Exemplary tissue targets may include liver, skeletal muscle, heart muscle, adipose deposits, kidney, lung, vascular endothelium, epithelial and/or hematopoietic cells and bone cells. A nucleic acid of the invention may be delivered to cells in vivo using methods such as direct injection of DNA, receptor-mediated DNA uptake, viral-mediated transfection or non-viral transfection and lipid based transfection, all of which may involve the use of gene therapy vectors. Direct injection has been used to introduce naked DNA into cells in vivo (see e.g., Acsadi et al. (1991) Nature 332:815-818; Wolff et al. (1990) Science 247:1465-1468). A delivery apparatus (e.g., a "gene gun") for injecting DNA into cells in vivo may be used. Such an apparatus may be commercially available (e.g., 15 from BioRad). Naked DNA may also be introduced into cells by complexing the DNA to a cation, such as polylysine, which is coupled to a ligand for a cell-surface receptor (see for example Wu, G. and Wu, C. H. (1988) J. Biol. Chem. 263:14621; Wilson el al. (1992) J. Biol. Chem. 267:963-967; and U.S. Pat. No. 5,166,320). Binding of the DNA-ligand complex to the receptor may facilitate uptake of the DNA by receptor-mediated endocytosis. A DNA-ligand complex linked to adenovirus capsids which disrupt endosomes, thereby releasing material into the cytoplasm, may be used to avoid degradation of the complex by intracellular lysosomes (see for example Curiel el al. (1991) Proc. Natl. Acad. Sci. USA 88:8850; Cristiano et al. (1993) Proc. Natl. Acad. Sci. USA 90:2122-2126). Defective retroviruses are well characterized for use as gene therapy vectors (for a review see Miller, A. D. (1990) Blood 76:271). 30 Protocols for producing recombinant retroviruses and for infecting cells in vitro or in vivo with such viruses can be found in Current Protocols in Molecular Biology, Ausubel, F. M. et al. (eds.) Greene Publishing Associates, (1989), Sections 9.10-9.14 and other standard laboratory manuals. Examples of suitable retroviruses include pLJ, pZIP, pWE and pEM which are well known to those skilled in the art. Examples of suitable packaging virus lines include .psi.Crip, .psi.Cre, .psi.2 and .psi.Am. Retroviruses have been used to introduce a variety of genes into many different cell types, including epithelial cells, endothelial cells, lymphocytes, myoblasts, hepatocytes, bone marrow cells, in vitro and/or in vivo (see for example Eglitis, et al. (1985) Science 230:1395-1398; Danos and Mulligan (1988) Proc. Natl. Acad. Sci. USA 85:6460-6464; Wilson et al. (1988) Proc. Natl. Acad. Sci. USA 85:3014-3018; Armentano et al. (1990) Proc. Natl. Acad. Sci. USA 87:61416145; Huber et al. (1991) Proc. Natl. Acad. Sci. USA 88:80398043; Ferry et al. (1991) Proc. Natl. Acad. Sci. USA 88:83778381; Chowdhury et al. (1991) Science 254:1802-1805; van Beusechem et al. (1992) Proc. Natl. Acad. Sci. USA 89:7640-7644; Kay et al. (1992) Human Gene Therapy 3:641-647; Dai et al. (1992) Proc. Natl. Acad. Sci. USA 89:10892-10895; Hwu et al. (1993) J. Immunol. 150:4104-4115; U.S. Pat. No. 4,868,116; U.S. Pat. No. 4,980,286; PCT Application WO 89/07136; PCT Application WO 89/02468; PCT Application WO 89/05345; and PCT Application WO 92/07573).

For use as a gene therapy vector, the genome of an adenovirus may be manipulated so that it includes an PTHrP nucleic acid, but is inactivated in terms of its ability to replicate in a normal lytic viral life cycle. See for example Berkner et al. (1988) BioTechniques 6:616; Rosenfeld et al.(1991) Science 252:431-434; and Rosenfeld et al. (1992) Cell 68:143-155. Suitable adenoviral vectors derived from the adenovirus strain Ad type 5 d1324 or other strains of adenovirus (e.g., Ad2, Ad3, Ad7 etc.) are well known to those skilled in the art. Recombinant adenoviruses are advantageous in that they do not require dividing cells to be effective gene delivery vehicles and can be used to infect a wide variety of cell types, including airway epithelium (Rosenfeld et al. (1992) cited supra), endothelial cells (Lemarchand et al. (1992) Proc. Natl. Acad. Sci. USA 89:6482-6486), hepatocytes (Herz and Gerard (1993) Proc. Natl. Acad. Sci. USA 90:28122816) and muscle cells (Quantin el al. (1992) Proc. Natl. Acad. Sci. USA 89 : 2581-2584).

Adeno-associated virus (AAV) may be used as a gene therapy vector for delivery of DNA for gene therapy purposes. AAV is a naturally occurring defective virus that requires another virus, such as an adenovirus or a herpes virus, as a helper virus for efficient replication and a productive life cycle (Muzyczka et al. Curr. Topics in Micro. and Immunol. (1992) 158:97-129). AAV may be used to integrate DNA into non-dividing cells (see for example Flotte et al. (1992) Am. J. Respir. Cell. Mol. Biol. 7:349-356; Samulski et al. (1989) J. Virol. 63:3822-3828; and McLaughlin et al. (1989) J. Virol. 62:19631973). An AAV vector such as that described in Tratschin et al. (1985) Mol. Cell. Biol. 5:3251-3260 may be used to introduce DNA into cells (see for example Hermonat et al. (1984) Proc. Natl. Acad. Sci. USA 81: 6466-6470; Tratschin et al. (1985) Mol. Cell. Biol. 4:2072-2081; Wondisford et al. (1988) Mol. Endocrinol. 2:32-39; Tratschin et al. (1984) J. Virol. 51:611619; and Flotte et al. (1993) J. Biol. Chem. 268:3781-3790). Lentiviral gene therapy vectors may also be adapted for use in 30 the invention.

General methods for gene therapy are known in the art, for example, U.S. Pat. No. 5,399,346 by Anderson et al. A biocompatible capsule delivering genetic material is described in PCT Publication WO95/05452 by Baetge et al. Methods of gene transfer into hematopoietic cells have also previously been reported (see Clapp, D. W. , et al., Blood 78: 1132-1139 (1991); Anderson, Science 288:627-9 (2000); and, Cavazzana-Calvo et al. , 288:669-72 (2000).

Further, a transgenic non- human mammal is provided to study bone disease and the mechanisms thereof. More particularly, a non-human transgenic mammal may be useful in studying the role of a PTHrP in an osteoblast specific manner, the non-human mammal having an disrupted/inactivated PTHrP gene (or portion thereof) specifically in osteoblast cells. The osteoblast cells of a non-human transgenic mammal of the present invention may be homozygous or heterozygous with respect to the disrupted PTHrP gene. Furthermore, a non- human transgenic mammal may be employed to screen compounds and/or agents for their effectiveness in modulating PTHrP expression and/or in treating, preventing or slowing the progression of bone disease. When patients requiring bone formation or repair are treated with a compound shown to increase PTHrP expression, PTHrP production by osteoblasts will be stimulated, resulting in bone formation and repair. Therefore, compounds identified in accordance with this embodiment of the present invention to modulate PTHrP expression may be used in the treatment of bone disease, bone breakdown, bone trauma, underdevelopment of bone and other conditions where PTHrP production is desired.

In an embodiment, somatic cells of the non-human mammal comprise PTHrP gene disruption means, for example capable of deleting/splicing out or inactivating a PTHrP gene, a portion thereof, or a nucleic acid sequence substantially identical in thereto, specifically osteoblast cells. "Specifically in osteoblast cells" as used herein refers to removal or inactivation in osteoblast cells with substantially no removal or inactivation in non-osteoblast cells. In an embodiment, the mammal is a rodent, in a further embodiment, a mouse.

In an embodiment, the above noted PTHrP gene disruption means comprises (a) a Cre recombinase gene osteoblast specific transcriptional regulatory region, such as a promoter; and (b) loxP sites which flank the PTHrP gene or a portion thereof. In an embodiment,the osteoblast-specific promoter is the type 1 collagen promoter.

As described in the Examples below, such animals may be used to study a number of PTHrP-dependent phenotypes in bone tissue, as well as having applications as described above. For example, such animals may be useful for the study of bone disease, bone formation, bone breakdown and bone trauma and for the discovery and development of therapeutics for bone disease. In particular, the transgenic non-human mammals of the present invention have particular application in the screening of potential therapeutic compounds and/or agents for effectiveness in treating, preventing or ameliorating bone disease and/or bone conditions. The transgenic non-human mammal of the invention is advantageous in this regard as it provides a viable animal amenable to study over longer experimental periods, and still provides a homozygous null PTHrP -/background in osteoblast cells, allowing the study of bone development and disease with respect to PTHrP function. For example, PTHrP flox/flox/ostCre mice will be treated with the experimental agents or vehicle alone for a period of one to two months and then sacrificed to have their skeletons examined by various techniques including BMD, histology, histomorphometry, immunohistochemistry etc. Increased bone density and none microarchitecture of experimental versus vehicle-treated animals will be considered significant.

Although various embodiments of the invention are disclosed herein, many adaptations and modifications may be made within the scope of the invention in accordance with the common general knowledge of those skilled in this art. Such modifications include the substitution of known equivalents for any aspect of the invention in order to achieve the same result in substantially the same way. Numeric ranges are inclusive of the numbers defining the range. In the claims, the word "comprising" is used as an open-ended term, substantially equivalent to the phrase "including, but not limited to".

The following examples are illustrative of various aspects of the invention, and do not limit the broad aspects of the invention as disclosed herein.

**EXAMPLES**

**Example 1: Analysis of PTHrP (+/-) heterozygous mice**

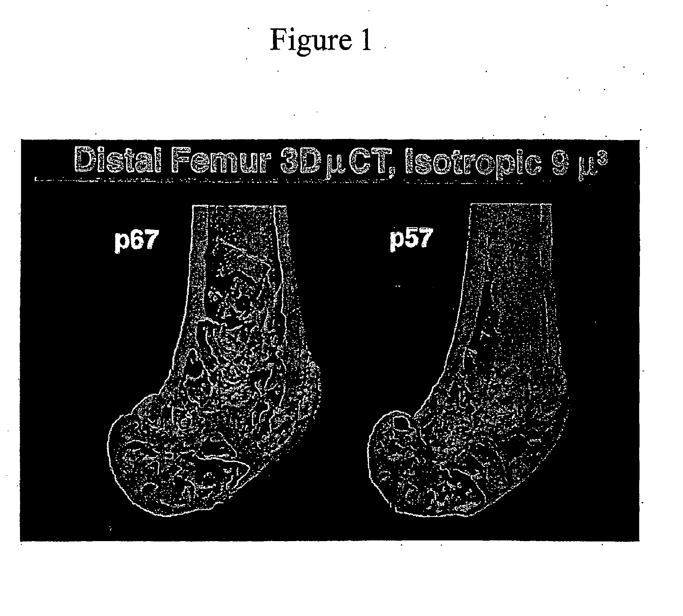

To assess the degree of bone loss in the PTHrP (+/-) animals, bones were removed from these animals and analysed using microCT. The bones were scanned on a $\mu$CT 20 system (Scanco USA, Inc. Wayne, PA) at a resolution of 18 $\mu$m$^3$. A set of images was obtained from each sample. Three-dimensional analysis was conducted on 10 manually selected volume of interest to calculate trabecular bone morphometric parameters. Figure 1 is a representative picture from the normal (left specimen) and heterozygous mice (right specimen) showing again the diminished content of trabecular bone in the mutant animals.

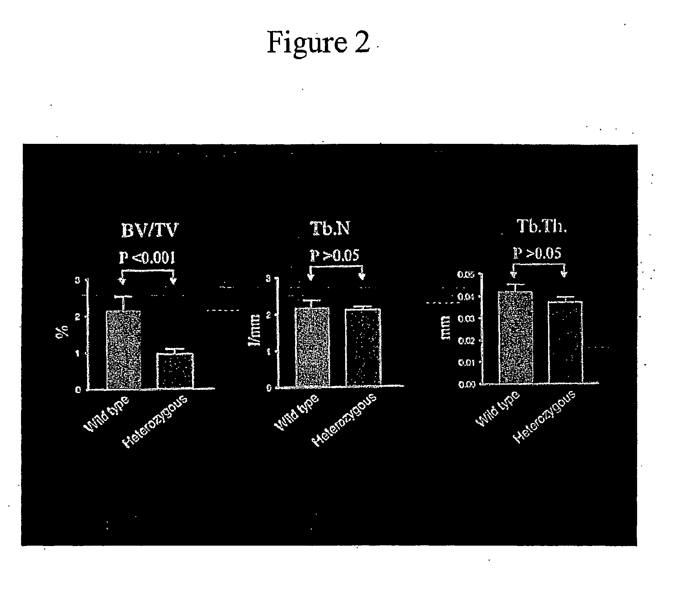

Figure 2 shows the measured bone volume (BV/TV), analysed as described above, which is decreased by more than 50% in the mutant animals while the number (Tb.N) and thickness (Tb.Th.) of bone trabecules was not altered.

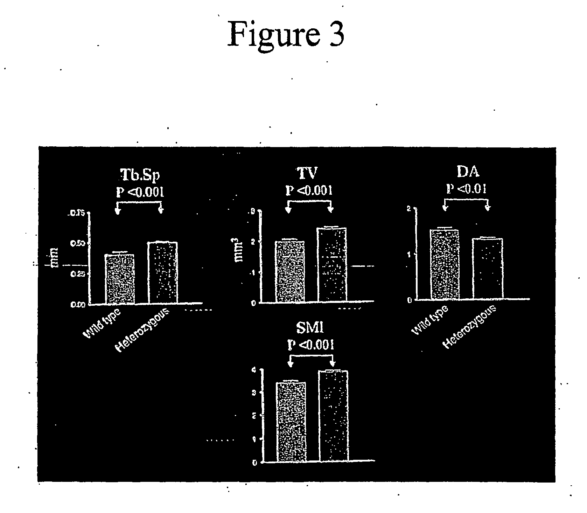

As shown in Figure 3, the spacing between trabecules was significantly increased, while other parameters such as DA (degree of anisotropy) and SMI (Structure Model Index), features that describe the 3-D architecture of the bone, strongly suggested the presence of osteoporotic changes in the mutant mice.

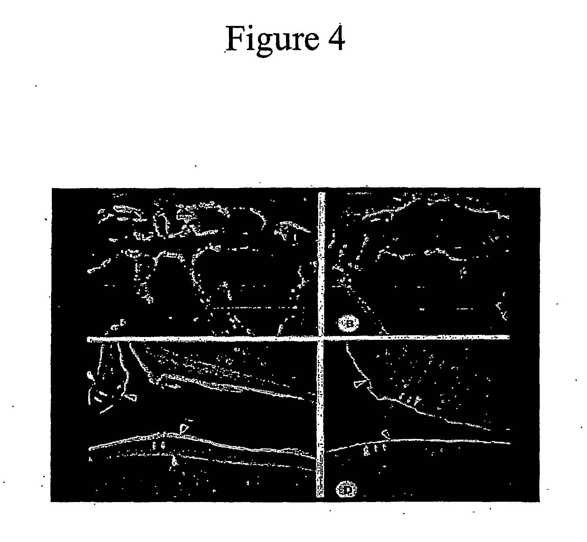

To ascertain the reason for the decreased amount of bone, mice were injected intraperitoneally with calcein (20 mg/kg body weight) and oxytetracycline (30 mg/kg body weight) and 4 days prior to sacrifice, respectively. One femur and one tibia per animal was removed and processed undecalcified in methylmethacrylate(a chemical that incorporates in newly deposited bone and gives off green fluorescent light), and 14 days later the same animals were injected with tetracycline (gives off orange red light). As shown in Figure 4, The distance between the two lines (Panels C and D) can be used to calculate the rate of bone formation, which is markedly diminished in the mutant mice.

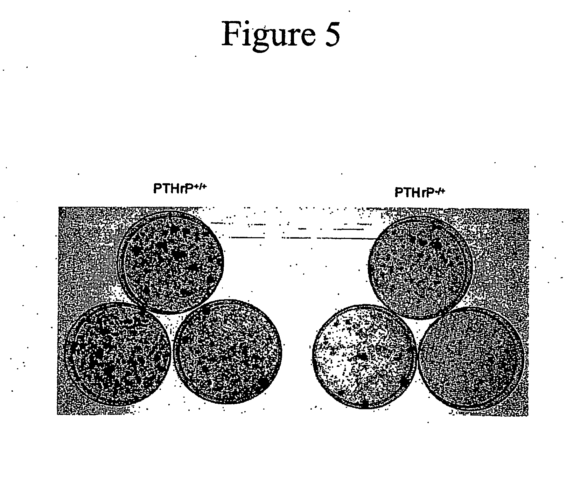

To evaluate the reason for the decreased bone formation in the PTHrP (+/-) mice, as shown in Figure 5, bone marrow from normal (left) and mutant (right) animals was extracted,cultured and induced to differentiate into osteoblasts (boneforming cells). Bone marrow was flushed from both ends with 1 ml a-MEM medium and cell cultures prepared. Adherent cells were recovered and plated at a density of 104 cells/35 mm dish in a-MEM containing loo fetal calf serum, antibiotics (100 ug/ml of pen G, 50 ug/ml of gentamycin, and 0.3 ug/ml of Fungizone), 50 ug/ml of ascorbic acid, 10-9 M dexamethasone and 10 mM Na-R-glycerophosphate to promote the formation of mineralized bone nodules. Medium was changed every 2-3 days, for 20 days. At the end of this period, cells were fixed and stained for alkaline phosphatase. Colonies were quantified using a dissecting microscope. Here, it is shown that the mutant marrow cannot differentiate into osteoblasts as readily as that of normal mice. Therefore, osteoblast differentiation is impaired.

## Example 2: Selective inactivation of PTHrP in osteoblasts in mice

In the heterozygous PTHrP (+/-) mice, the PTHrP gene is removed from every cell in the body, including osteoblasts and hondrocytes, the cartilage-forming cells. Since bone is derived from cartilage, it is possible that the observations were a reflection of improper cartilage formation per se rather than impaired bone formation.

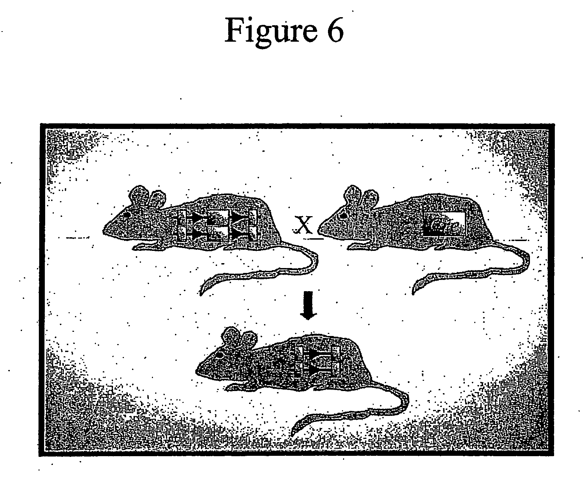

To rule out this possibility, mice were generated (shown schematically in Figure 6) that are missing PTHrP only from osteoblasts using the Cre-LoxP system for which two mice are needed: one contains the PTHrP gene flanked by LoxP sequences and the second, is a transgenic mouse expressing Cre recombinase under an osteoblast-specific promoter (type 1 collagen) (He et al.(2001) Endocrinology 142(5):2070 -7). When mated, the PTHrP gene is removed only from osteoblasts. All other cells will be normal. Specifically, mice carrying the site-specific recombinase gene (cre) under the control of the type I collagen (Col I) promoter were crossed with PTHrP floxed mice (PTHrp$^{flox/flox}$) in which the exon 4 of both PTHrP alleles was flanked by loxP sites to generate mice with osteoblasts lacking PTHrP expression (PTHrP$^{flox/flox}$; cre$^{col I}$).

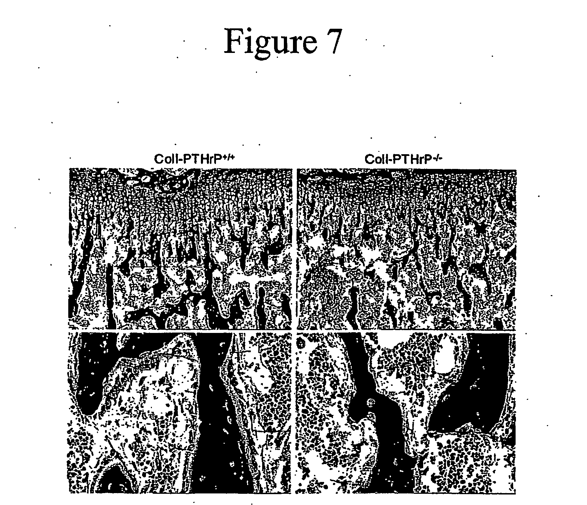

As shown in Figure 7, osteoblasts developed premature as shown in the top panels trabecular bone). The panel at trabecule (black color) lined functional osteoblasts. In contrast, mutant mice (lower right) does not display a significant number of healthy osteoblasts. Therefore, absence of PTHrP from osteoblasts leads to poor osteoblast function and diminished bone formation.

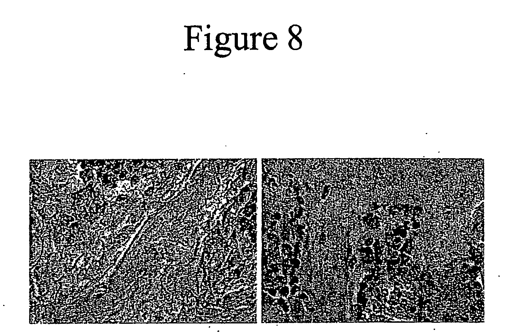

As shown in Figure 8, osteoblasts missing PTHrP were not very evident in bone because they died by apoptosis. As shown in the right panel, staining of osteoblasts with a specific assay for apoptotic nuclei is very much evident in the mutant animals missing PTHrP from osteoporosis (2 months of age) (black color corresponds to the bottom left shows a normal up by plump, healthy-looking, trabecular bone from specimens (red color, right panel) but not in the wild type specimens (left panel).

These findings indicate that PTHrP is critical for normal osteoblast development, since its absence leads to decreased osteoblast generation and early apoptotic death. The final result is decreased bone formation and the premature development of osteoporosis.

It was further determined that serum calcium, PTH and 1,25 dihydroxyvitamin D3 levels and parathyroid gland size were normal in 6 week PTHrP$^{flox/flox}$ ; cre$^{sol I}$ mice as compared to the wild type (PTHrP$^{flox/flox}$) control littermates. Bone density of femurs and tibiae on the other hand were found to be decreased by 6.33 % and 9.00% in 6 week PTHrPflox/flox; cre Col I mice as compared to wild type mice as measured by PIXImus densitometer. Trabecular bone volume was also affected where femurs and tibiae were decreased by 33.33 % and 35.50 % respectively in newborn PTHrP$^{flox/flox}$; cre $^{col I}$ mice and 33.90 % and 47.00 % in 6 week PTHrP$^{flox/flox}$; cre $^{col I}$ mice in comparison to their wild type littermates. Histomorphometric analysis revealed that osteoblast number and surface, osteoid thickness, trabecular thickness all were reduced significantly in 6 week PTHrP$^{flox/flox}$; cre $^{col I}$ mice compared to wild type mice.

This was further substantiated by immunohistochemistry as assessed by measurements of positive areas for type I collagen, osteocalcin, and osteopontin staining in the metaphysis of femurs of 6 week PTHrP$^{-/flox}$; cre $^{col I}$ mice compared to their wild type counterparts. Bone formation rates were also reduced as demonstrated by double calcein label histomorphometry when compared to 6 week wild type mice. Furthermore, osteoblast 30 number and surface were also reduced significantly in 6 week PTHrP$^{-/flox}$; cre $^{col I}$ mice compared to wild type mice. These results therefore suggest that PTHrP exerts its anabolic action at least in part by an autocrine manner and may regulate or stimulate osteoblast formation and recruitment.

The above protocol was adapted to generate heterozygous osteoblast-specific PTHrP disrupted animals, in accordance with the present invention.

## Example 3: Comparison of PTHrP genetic sequences between osteoporitic and healthy subjects

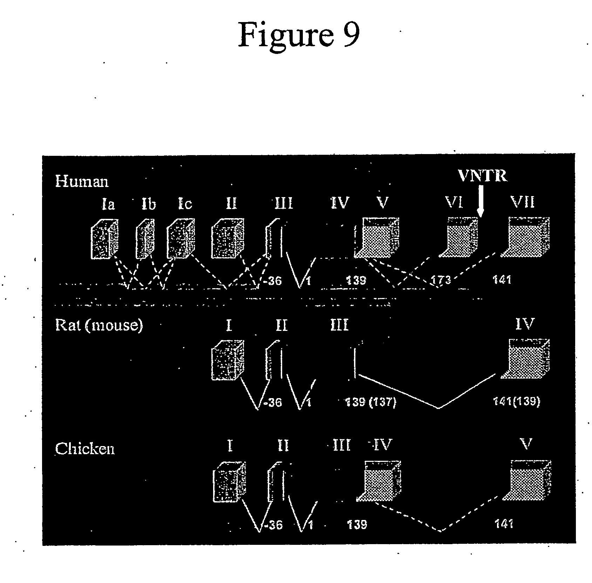

The human PTHrP gene structure is depicted among others in Figure 9, and is further disclosed by Yasuda T. et al. in J Biol Chem. 1989 May 5;264(13):7720-5). The arrow points to the region of DNA that encompasses a VNTR, a Variable Number of Tandem Repeats, that has been previously described but no functionality was ascribed to it (Pausova Z. et al. Genomics. 1993 17(1):243-4).

As illustrated in Figure 10, the VNTR-containing sequence can be amplified from genomic DNA using PCR with the oligonucleotide primers for the regions underlined. The number of tandem repeats (G/ATATATATA)n gives rise to various lengths of amplified DNA depending on the number of these repeats contained in an individual's DNA.

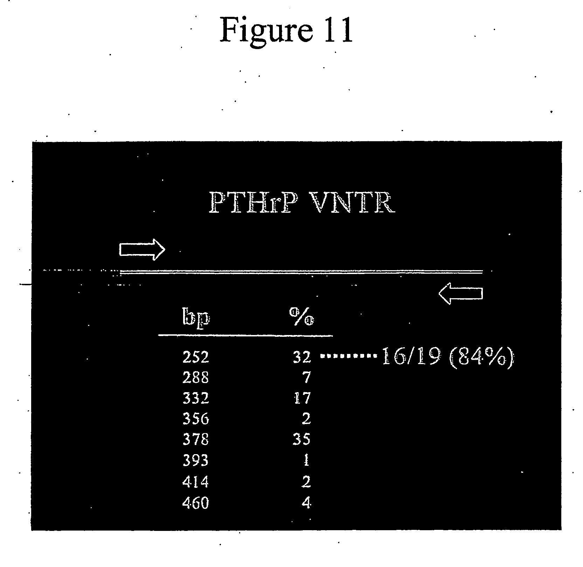

As shown in Figure 11, the prevalence of the various VNTRs in the general population, ranges from 252 base pairs (bp) to 460 bp in length. Clearly, the 252 bp and the 378 bp VNTRs are the most common, although several less frequent ones are also indicated.

The PTHrP VNTR region in 19 osteoporotic males subjects was then examined. These patients had the diagnosis of idiopathic osteoporosis, i.e. likely due to a genetic etiology. As shown in Figure 11, in this sample of patients, 16/19 (84%) had the 252 bp allele, a frequency much higher than that seen in the general population (32%), indicating that short VNTRs may be important in determining a higher risk for osteoporosis in a specific individual.

## MATERIALS AND METHODS

STUDY DESIGN

Patients presenting to the osteoporosis clinic with the finding of a low or normal bone mass density (BMD) were invited to enter the study and a consent form will be signed. The diagnosis of osteoporosis due to genetic etiology will be made upon the presentation of a positive family history and exclusion of secondary etiologies. Lumbar spine (L2-4), femoral neck, and trochanter T- (BMD compared to young adults) and Z- (BMD compared to age matched controls) scores will be recorded. Also baseline characteristics such as age, height, weight, calcium intake, 25(OH)vitamin D levels, will be obtained. A blood sample (10 cc) will be collected from every patient. DNA will be isolated from the blood cells and assayed by Polymerase Chain Reaction (PCR) for the PTHrP VNTR. Amplified DNA fragments will then be examined for their length using polyacrylamide gel electrophoresis.

Patient's confidentiality will be respected, under the limits of the law. If they wish, patients can terminate their participation in this study, at any time. There will be no costs, nor payments, for patients participating in this study.

EXCLUSION CRITERIA

Patients with history of bone disease or those that are taking medications that affect bone turnover will be excluded from the study. Patients that are already being treated for osteoporosis will not be enrolled in the study.

RISKS FOR THE PATIENTS

The risks related to this study are minimal. They are associated with taking blood and include pain, bruises, or, rarely, fainting.

BENEFITS FOR THE PATIENTS

The patients will be checked for osteoporosis and will receive appropriate follow up and treatment in case of a decrease in their BMD.

RELEVANCE

The PTHrP VNTR genetic marker will be used as a diagnostic tool in the assessment of individuals at risk of developing osteoporosis and osteoporotic fractures. We already know that the BMD T-score value of -2.5, which is widely used as a treatment threshold for osteoporosis, identifies only a small proportion of individuals in the community who actually suffer fractures. Genetic markers of bone fragility or bone loss could be used prior to and alongside BMD measurements to help target early preventative therapies to those individuals who are at risk of fracture.

STATISTICAL ANALYSIS

This is an exploratory study of the predictive value of VNTR length on BMD. The length of VNTR in each patient will be recorded. As well, 2 markers of BMD will be recorded namely: Lumbar spine and femoral neck.

The statistical objective will be to show that there is a statistically significant relationship between VNTR length and two predictor variables of BMD. The outcome measures are:

Response: Y=VNTR length, a continuous measure

Predictors: X1=Lumbar Spine, X2= Femoral neck, X3=Trochanter, all three as continuous variables.

This relationship will be controlled for baseline characteristics such as age, height, weight, calcium intake and 25(OH) vitamin D levels. Sex will be another control variable but with a nesting of the level of osteoporosis.

There will be a total of 80 participants in this study: two groups of males with 20 participants per group: Normal and Osteoporotic; two groups of premenopausal females with 20 participants per group: Normal and Osteoporotic

The following analysis of covariance analyses will be performed:

```
Y= X1 + X2 + X3 + age + height + weight +calcium + vitamin
+ Sex + Osteoporosis level (sex)
```

All baseline characteristics that are not significant will be dropped from the analysis and subsequent conclusions based on the reduced model. All analyses will be performed in SAS version 8.12. Statistical significance of the regression coefficients of X1, X2, and X3 will be indicative of probable predictive value, to be confirmed in further studies.

**Example 4: Development of an osteoblast-specific oligonucleotide**

We have recently shown that the following oligonucleotide [SEQ ID NO. 1] :

5' -TATATACGTATATATATATACGTATATATATACGTA - 3'

containing the GTATATATA sequence of the VNTR is bound by nuclear proteins when the oligonucleotide was incubated with cell nuclear extracts. Specifically, when we incubated the VNTR oligonucleotide with COS cell nuclear extracts (non-osteogenic cell line) there was no specific binding of any nuclear proteins. However, when we incubated the VNTR oligonucleotide with ROS cell nuclear extracts (osteogenic cell line) there was specific binding of many nuclear proteins.

The precise nature of these protein(s) remains to be determined. However, this particular region is critical for PTHrP expression and the protein(s) that are binding to the oligonucleotide sequence [SEQ ID NO. 1] could lead to the discovery of novel therapeutics and diagnostic markers of bone disease.

The invention also includes nucleic acids that hybridize under moderately stringent, or preferably stringent hybridization conditions to all or a portion of the oligonucleotide sequence represented by [SEQ ID NO:1] or its complement. Stringent conditions are sequence dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993) (Elsevier Science, Inc., New York). Generally, stringent conditions are selected to be about 5-10°C lower than the thermal melting point (Tm) for the specific sequence at a defined ionic strength and pH. The Tm is the temperature (under defined ionic strength and pH) at which 50% of the target sequence hybridizes to a perfectly matched probe. Typically, stringent conditions will be those in which the salt concentration is less than about 1.0 M sodium ion, typically about 0.01 to 1.0 M sodium ion concentration (or other salts) at pH 7.0 to 8.3 and the temperature is at least about 30°C. for short probes (e.g., 10 to 50 nucleotides) and at least about 60 °C for long probes (e.g., greater than 50 nucleotides). Stringent conditions may also be achieved with the addition of destabilizing agents such as formamide. For selective or specific hybridization, a positive signal is at least two times background, preferably 10 times background hybridization. Exemplary stringent hybridization conditions can be as following: 50% formamide, 5x SSC, and 1% SDS, incubating at 42 °C or, 5x SSC, 1% SDS, incubating at 65 °C, with wash in 0.2x SSC, and 0.1% SDS at 65°C. For the purpose of the invention, suitable "moderately stringent conditions" include, for example, prewashing in a solution of 5x SSC, 0.5% SDS, 1.0 mM EDTA (pH 8.0), hybridizing at 50°C - 65°C., 5x SSC overnight, followed by washing twice at 65°C. for 20 minutes with each of 0.5x and 0.2x SSC (containing 0.1% SDS). Such hybridizing DNA sequences are also within the scope of this invention.

The embodiment(s) of the invention described above is(are) intended to be exemplary only. The scope of the invention is therefore intended to be limited solely by the scope of the appended claims.

## Claims

1. A method of diagnosing bone disease in an individual, said method comprising:

   a) screening for a variable number tandem repeat (VNTR) region within a parathyroid hormone related peptide (PTHrP) gene in a biological sample from said individual;
   b) comparing the length of the VNTR region of said individual with VNTR region lengths of PTHrP genes within a population; and
   c) diagnosing said individual with respect to bone disease based on the results obtained in step (b), wherein a respectively shorter length of said VNTR region is correlated with a diagnosis for bone disease or a predisposition thereto.

2. The method of claim 1 wherein the VNTR region is between 252 to 460 base pairs in length.

3. The method of claim 1 or 2, wherein said VNTR region includes a variable number of tandem repeats.

4. The method of any one of claims 1 to 3, wherein said VNTR region is 252bp, 288bp, 332bp, 356bp, 378bp, 393bp, 414bp or 460bp in length.

5. The method of any one of claims 1 to 4, wherein the VNTR region in the PTHrP gene comprises 9 nucleotide repeats; wherein said 9 nucleotide repeats are selected from the group consisting of GTATATATA and ATATATATA.

6. The method of any one of claims 1 to 5, wherein the screening step comprises detecting alleles having a variable number of 9 nucleotide repeats in the PTHrP gene; wherein said 9 nucleotide repeats are selected from the group consisting of GTATATATA and ATATATATA.

7. The method of any one of claims 1 to 6, wherein the bone disease is selected from the group consisting of osteoporosis, osteomalacia, osteopenia, osteopetrosis, Paget's disease and renal osteodystrophy.

8. The method of any one of claims 1 to 7, wherein the screening step comprises amplification of a DNA sequence from the VNTR region.

9. The use of a length of a variable number tandem repeat (VNTR) region within a parathyroid hormone related peptide (PTHrP) gene of an individual as an indicator of bone disease and/or a predisposition thereto, wherein a shorter length of said VNTR region is correlated with a diagnosis for bone disease or a predisposition thereto.

10. The use according to claim 9, wherein the VNTR region comprises a variable number of 9 nucleotide repeats in the PTHrP gene, wherein said 9 nucleotide repeats are selected from the group consisting of GTATATATA and ATATATATA.

**11.** The use according to claim 9 or 10, wherein the VNTR region is between 252 to 460 base pairs in length.

**12.** The use according to any one of claims 9 to 11, wherein the VNTR region is 252bp, 288bp, 332bp, 356bp, 378bp, 393bp, 414bp and 460bp in length.

**13.** A method of diagnosing a susceptibility for bone disease in an individual; said method comprising:

a) screening for a variable number tandem repeat (VNTR) region within a parathyroid hormone related peptide (PTHrP) gene in a biological sample from said individual;
b) comparing the length of the VNTR region of said individual with VNTR region lengths of PTHrP genes within a population; and
c) diagnosing said individual with respect to bone disease based on the results obtained in step (b), wherein a respectively shorter length of said VNTR region is correlated with a susceptibility for bone disease.

**14.** The method of claim 13, wherein said VNTR region is between 252 to 460 base pairs in length.

**15.** The method of claim 13 or 14, wherein said VNTR region includes a variable number of tandem repeats.

**16.** The method of any one of claims 13 to 15, wherein the VNTR region is 252bp, 288bp, 332bp, 356bp, 378bp, 393bp, 414bp or 460bp in length.

**17.** The method of any one of claims 13 to 16, wherein the VNTR region in the PTHrP gene comprises 9 nucleotide repeats; wherein said 9 nucleotide repeats are selected from the group consisting of GTATATATA and ATATATATA.

**18.** The method of any one of claims 13 to 17, wherein the screening step comprises detecting alleles having a variable number of 9 nucleotide repeats in the PTHrP gene; wherein said 9 nucleotide repeats are selected from the group consisting of GTATATATA and ATATATATA.

**19.** The method of any one of claims 13 to 18, wherein the screening step comprises amplification of a DNA sequence from the VNTR region.

**20.** A kit for use in providing a diagnosis and/or prediction of bone disease in an individual, the kit comprising:

means for screening for a variable number tandem repeat (VNTR) region within a parathyroid hormone related peptide (PTHrP) gene in a biological sample from said individual;
means for comparing the length of the VNTR region of said individual with VNTR region lengths of PTHrP genes within a population; and
instructions for correlating a diagnosis and/or prediction of bone disease with the results obtained from the VNTR region length comparison, whereby a respectively shorter length of said VNTR region is correlated with a diagnosis for bone disease or a predisposition thereto.

**21.** The kit of claim 20, further comprising means for measuring the length of said VNTR region of the PTHrP gene.

**22.** The kit of claim 20 or 21, further comprising means for amplifying the VNTR region of said PTHrP gene.

**23.** The kit of any one of claims 20 to 22, wherein said means for screening comprise probes specific for the VNTR region of said PTHrP gene.

**24.** The kit of claim 23, wherein said probes are specific for a 9 nucleotide repeat selected from the group consisting of GTATATATA and ATATATATA.

**25.** An isolated single-stranded nucleic acid having a nucleotide sequence consisting of the sequence of SEQ ID No: 1, or the complement thereof.

**26.** Use of the single-stranded nucleic acid of claim 25, as a probe for diagnosing bone disease and/or a predisposition thereto.

**27.** Use of the single-stranded nucleic acid of claim 25, for identifying candidate compounds having the ability to modulate PTHrP expression.

28. Use of the single-stranded nucleic acid of claim 25, for identifying a protein that modulates PTHrP expression *in vivo.*

**Patentansprüche**

1. Verfahren zur Diagnostizierung von Knochenerkrankungen bei einer Person, wobei das Verfahren umfasst:

   a) Screening auf eine Variable Number Tandem Repeat (VNTR)-Region in einem Parathormon-verwandten Peptid (Parathyroid Hormone related Peptide; PTHrP)-Gen in einer biologischen Probe dieser Person;
   b) Vergleichen der Länge der VNTR-Region der Person mit den Längen der VNTR-Region von PTHrP-Genen in einer Population; und
   c) Diagnostizieren im Hinblick auf eine Knochenerkrankung bei dieser Person auf der Basis der in Schritt b) erhaltenen Ergebnisse, wobei eine kürzere Länge der VNTR-Region mit einer Diagnose für eine Knochenerkrankung oder eine Prädisposition dafür korreliert.

2. Verfahren nach Anspruch 1, wobei die Länge der VNTR-Region im Bereich von 252 bis 460 Basenpaare liegt.

3. Verfahren nach Anspruch 1 oder 2, wobei die VNTR-Region eine variable Anzahl von Tandem-Repeats umfasst.

4. Verfahren nach einem der Ansprüche 1 bis 3, wobei die Länge der VNTR-Region 252 bp, 288 bp, 332 bp, 356 bp, 378 bp, 393 bp, 414 bp oder 460 bp ist.

5. Verfahren nach einem der Ansprüche 1 bis 4, wobei die VNTR-Region in dem PTHrP-Gen 9-Nucleotid-Repeats enthält; wobei die 9-Nukleotid-Repeats unter GTATATATA und ATATATATA ausgewählt sind.

6. Verfahren nach einem der Ansprüche 1 bis 5, wobei der Screening-Schritt den Nachweis von Allelen mit einer variablen Anzahl von 9-Nukleotid-Repeats in dem PTHrP-Gen umfasst; wobei die 9-Nukleotid-Repeats unter GTATATATA und ATATATATA ausgewählt sind.

7. Verfahren nach einem der Ansprüche 1 bis 6, wobei die Knochenerkrankung unter Osteoporose, Osteomalazie, Osteopenia, Osteopetrosis, Morbus Paget und renaler Osteodystrophie ausgewählt ist.

8. Verfahren nach einem der Ansprüche 1 bis 7, wobei der Screening-Step die Amplifizierung einer DNA-Sequenz aus der VNTR-Region umfasst.

9. Verwendung einer Länge einer Variable-Number-Tandem-Repeat (VNTR)-Region in einem Parathormon-änlichen-Peptid (Parathyroid Hormone related Peptide; PTHrP)-Gen einer Person als Indikator für eine Knochenerkrankung und/oder eine Prädisposition dafür, wobei eine kürzere Länge der VNTR-Region mit einer Diagnose für eine Knochenerkrankung oder eine Prädisposition dafür korreliert.

10. Verwendung nach Anspruch 9, wobei die VNTR-Region eine variable Anzahl von 9-Nucleotid-Repeats in dem PTHrP-Gen umfasst, wobei die 9-Nucleotid-Repeats ausgewählt sind unter GTATATATA und ATATATATA.

11. Verwendung nach Anspruch 9 oder 10, wobei die Länge der VNTR-Region im Bereich von 252 bis 460 liegt.

12. Verwendung nach einem der Ansprüche 9 bis 11, wobei die Länge der VNTR-Region 252 bp, 288 bp, 332 bp, 356 bp, 378 bp, 393 bp, 414 bp und 460 bp ist.

13. Verfahren zur Diagnostizierung der Anfälligkeit einer Person für eine Knochenerkrankung; wobei das Verfahren umfasst:

   a) Screening auf eine Variable-Number-Tandem-Repeat (VNTR)-Region in einem Parathormon-ähnlichen-Peptid (Paratyroid Hormone related Peptide; PTHrP)-Gen in einer biologischen Probe dieser Person;
   b) Vergleichen der Länge der VNTR-Region der Person mit den Längen der VNTR-Region von PTHrP-Genen in einer Population; und
   c) Diagnostizieren im Hinblick auf eine Knochenerkrankung bei dieser Person auf der Basis der in Schritt b) erhaltenen Ergebnisse, wobei die kürzere Länge der VNTR-Region mit der Anfälligkeit für eine Knochenerkrankung korreliert.

14. Verfahren nach Anspruch 13, wobei die Länge der VNTR-Region im Bereich von 252 bis 460 Basenpaare liegt.

15. Verfahren nach Anspruch 13 oder 14, wobei die VNTR-Region eine variable Anzahl von Tandem-Repeats umfasst.

16. Verfahren nach einem der Ansprüche 13 bis 15, wobei die Länge der VNTR-Region 252 bp, 288 bp, 332 bp, 356 bp, 378 bp, 393 bp, 414 bp oder 460 bp ist.

17. Verfahren nach einem der Ansprüche 13 bis 16, wobei die VNTR-Region in dem PTHrP-Gen 9-Nukleotid-Repeats enthält; wobei die 9-Nukleotid-Repeats unter GTATATATA und ATATATATA ausgewählt sind.

18. Verfahren nach einem der Ansprüche 13 bis 17, wobei der Screening-Schritt den Nachweis von Allelen mit einer variablen Anzahl von 9-Nukleotid-Repeats in dem PTHrP-Gen umfasst; wobei die 9-Nukleotid-Repeats unter GTATATATA und ATATATATA ausgewählt sind.

19. Verfahren nach einem der Ansprüche 13 bis 18, wobei der Screening-Schritt die Amplifizierung einer DNA-Sequenz aus der VNTR-Region umfasst.

20. Kit für die Verwendung bei einer Diagnose und/oder Prognose einer Knochenerkrankung bei einer Person, wobei das Kit umfasst:

   - Mittel zum Screening auf eine Variable-Number-Tandem-Repeat (VNTR)-Region in einem Parathormon-ähnlichen-Peptid (PTHrP)-Gen in einer biologischen Probe der Person;
   - Mittel zum Vergleichen der Länge der VNTR-Region der Person mit den Längen der VNTR-Region von PTHrP-Genen in einer Population; und
   - Instruktionen für die Korrelation der Ergebnisse, die aus dem Vergleich der VTNR-Region-Länge erhalten werden, mit einer Diagnose und/oder Prognose einer Knochenerkrankung, wobei eine entsprechende kürzere Länge der VTNR-Region mit einer Diagnose für eine Knochenerkrankung oder einer Prädisposition dafür korreliert.

21. Kit nach Anspruch 20, das ferner Mittel zur Messung der Länge dieser VNTR-Region des PTHrP-Gens enthält.

22. Kit nach Anspruch 20 oder 21, das ferner Mittel für die Amplifizierung der VNTR-Region des PTHrP-Gens enthält.

23. Kit nach einem der Ansprüche 20 bis 22, wobei die Mittel zum Screening spezifische Sonden für die VNTR-Region des PTHrP-Gens umfassen.

24. Kit nach Anspruch 23, wobei die Sonden spezifisch für einen 9-Nucleotid-Repeat sind, der unter GTATATATA und ATATATATA ausgewählt ist.

25. Isolierte einsträngige Nucleinsäure mit einer Nukleotid-Sequenz, die aus der Sequenz von SEQ ID NO:1 oder ihrem Komplement besteht.

26. Verwendung der einsträngigen Nucleinsäure nach Anspruch 25 als Sonde für die Diagnostizierung einer Knochenerkrankung und/oder einer Prädisposition dafür.

27. Verwendung der einsträngigen Nucleinsäure nach Anspruch 25 für die Identifizierung von möglichen Verbindungen mit der Fähigkeit, die PTHrP-Expression zu verändern.

28. Verwendung der einsträngigen Nucleinsäure nach Anspruch 25 für die Identifizierung eines Proteins, das in vivo die PTHrP-Expression verändert.

**Revendications**

1. Une méthode de diagnostic d'une maladie osseuse chez un individu, ladite méthode comprenant:

a) La calibrage d'une zone de répétitions en tandem en nombre variable (VNTR) à l'intérieur d'un gène du peptide apparenté à l'hormone parathyroïdienne (PTHrP) dans un échantillon biologique dudit individu;

b) la comparaison de la longueur de la zone VNTR dudit individu avec les longueurs des zones VNTR des gènes PTHrP au sein d'une population; et

c) le diagnostic dudit individu en ce qui concerne une maladie osseuse sur la base des résultats obtenus à l'étape b), dans lequel une longueur respectivement plus courte de ladite zone VNTR est corrélée avec un diagnostic d'une maladie osseuse ou une prédisposition à celle-ci.

2. La méthode de la revendication 1, dans laquelle la zone VNTR a une longueur située entre 252 et 460 paires de base.

3. La méthode de la revendication 1 ou 2, dans laquelle ladite zone VNTR inclut un nombre variable de répétitions en tandem.

4. La méthode de l'une quelconque des revendications 1 à 3, dans laquelle ladite zone VNTR a une longueur de 252bp, 288bp, 332bp, 356bp, 378bp, 393bp, 414bp ou 460bp.

5. La méthode de l'une quelconque des revendications 1 à 4, dans laquelle la zone VNTR dans le gène PTHrP comprend des répétitions de 9 nucléotides; dans laquelle lesdites répétitions de 9 nucléotides sont sélectionnées dans le groupe composé de GTATATATA et ATATATATA.

6. La méthode de l'une quelconque des revendications 1 à 5, dans laquelle l'étape de calibrage comprend la détection des allèles ayant un nombre variable de répétitions de 9 nucléotides dans le gène PTHrP; dans laquelle lesdites répétitions de 9 nucléotides sont sélectionnées dans le groupe composé de GTATATATA et ATATATATA.

7. La méthode de l'une quelconque des revendications 1 à 6, dans laquelle la maladie osseuse est sélectionnée parmi le groupe composé de l'ostéoporose, l'ostéomalacie, l'ostéopénie, l'ostéopétrose, la maladie de Paget et l'ostéodystrophie rénale.

8. La méthode de l'une quelconque des revendications 1 à 7, dans laquelle l'étape de calibrage comprend l'amplification d'une séquence ADN à partir de la zone VNTR.

9. L'utilisation d'une longueur d'une zone de répétitions en tandem en nombre variable (VNTR) à l'intérieur d'un gène du peptide apparenté à l'hormone parathyroïdienne (PTHrP) d'un individu comme indicateur d'une maladie osseuse et/ou d'une prédisposition à celle-ci, dans laquelle une longueur plus courte de ladite zone VNTR est corrélée avec un diagnostic d'une maladie osseuse ou une prédisposition à celle-ci.

10. L'utilisation selon la revendication 9, dans laquelle la zone VNTR comprend un nombre variable de répétitions de 9 nucléotides dans le gène PTHrP, dans laquelle lesdites répétitions de 9 nucléotides sont sélectionnées dans le groupe composé de GTATATATA et ATATATATA.

11. L'utilisation selon la revendication 9 ou 10, dans laquelle la zone VNTR a une longueur située entre 252 et 460 paires de base.

12. L'utilisation selon l'une quelconque des revendications 9 à 11, dans laquelle la zone VNTR a une longueur de 252bp, 288bp, 332bp, 356bp, 378bp, 393bp, 414bp ou 460bp.

13. Méthode de diagnostic d'une prédisposition à une maladie osseuse chez un individu; ladite méthode comprenant:

a) le calibrage pour une zone de répétitions en tandem en nombre variable (VNTR) à l'intérieur d'un gène du peptide apparenté à l'hormone parathyroïdienne (PTHrP) dans un échantillon biologique dudit individu;

b) la comparaison de la longueur de la zone VNTR dudit individu avec les longueurs des zones VNTR des gènes PTHrP au sein d'une population; et

c) le diagnostic dudit individu en ce qui concerne une maladie osseuse sur la base des résultats obtenus à l'étape b), dans lequel une longueur respectivement plus courte de ladite zone VNTR est corrélée avec une prédisposition à une maladie osseuse.

14. La méthode de la revendication 13, dans laquelle ladite zone VNTR a une longueur située entre 252 et 460 paires de base.

**15.** La méthode de la revendication 13 ou 14, dans laquelle ladite zone VNTR inclut un nombre variable de répétitions en tandem.

**16.** La méthode de l'une quelconque des revendications 13 à 15, dans laquelle la zone VNTR a une longueur de 252bp, 288bp, 332bp, 356bp, 378bp, 393bp, 414bp ou 460bp.

**17.** La méthode de l'une quelconque des revendications 13 à 16, dans laquelle la zone VNTR dans le gène PTHrP comprend des répétitions de 9 nucléotides; dans laquelle lesdites répétitions de 9 nucléotides sont sélectionnées dans le groupe composé de GTATATATA et ATATATATA.

**18.** La méthode de l'une quelconque des revendications 13 à 17, dans laquelle l'étape de calibrage comprend la détection des allèles ayant un nombre variable de répétitions de 9 nucléotides dans le gène PTHrP; dans laquelle lesdites répétitions de 9 nucléotides sont sélectionnées dans le groupe composé de GTATATATA et ATATATATA.

**19.** La méthode de l'une quelconque des revendications 13 à 18, dans laquelle l'étape de calibrage comprend l'amplification d'une séquence ADN à partir de la zone VNTR.

**20.** Kit devant être utilisé pour fournir un diagnostic et/ou une prévision d'une maladie osseuse chez un individu, le kit comprenant:

un moyen de calibrage pour une zone de répétitions en tandem en nombre variable (VNTR) à l'intérieur d'un gène du peptide apparenté à l'hormone parathyroïdienne (PTHrP) dans un échantillon biologique dudit individu; un moyen de comparaison de la longueur de la zone VNTR dudit individu avec les longueurs des zones VNTR des gènes PTHrP au sein d'une population; et des instructions pour la corrélation d'un diagnostic et/ou d'une prévision d'une maladie osseuse avec les résultats obtenus à partir de la comparaison de la longueur de zone VNTR, ainsi une longueur respectivement plus courte de ladite zone VNTR est corrélée avec un diagnostic d'une maladie osseuse ou une prédisposition à celle-ci.

**21.** Le kit de la revendication 20, comprenant en outre un moyen permettant de mesurer la longueur de ladite zone VNTR du gène PTHrP.

**22.** Le kit de la revendication 20 ou 21, comprenant en outre un moyen permettant d'amplifier la zone VNTR dudit gène PTHrP.

**23.** Le kit de l'une quelconque des revendications 20 à 22, dans lequel ledit moyen de calibrage comprend des sondes spécifiques pour la zone VNTR dudit gène PTHrP.

**24.** Le kit de la revendication 23, dans lequel lesdites sondes sont spécifiques pour une répétition de 9 nucléotides sélectionnée dans le groupe composé de GTATATATA et ATATATATA.

**25.** Acide nucléique monocaténaire isolé ayant une séquence de nucléotides composée de la séquence dont le numéro d'identification est 1 ou du complément de celle-ci.

**26.** Utilisation de l'acide nucléique monocaténaire de la revendication 25 comme une sonde pour diagnostiquer une maladie osseuse et/ ou une prédisposition à celle-ci.

**27.** Utilisation de l'acide nucléique monocaténaire de la revendication 25 pour identifier des composés candidats ayant la capacité de moduler l'expression du PTHrP.

**28.** Utilisation de l'acide nucléique monocaténaire de la revendication 25 pour identifier une protéine qui module l'expression du PTHrP in vivo.

# Figure 1

Distal Femur 3DμCT, Isotropic 9 μ³

p67  p57

# Figure 2

# Figure 3

# Figure 4

# Figure 5

PTHrP+/+            PTHrP-/+

# Figure 6

# Figure 7

Coll-PTHrP+/+        Coll-PTHrP-/-

# Figure 8

# Figure 9

# Figure 10

PTHrP VNTR

GACCTAGTTCTGATTGTATCCTCTACCTATCATCTATCTATTTATAT
CTATCTATCTATCTAGGGGGATATGTTTCTATGAGGCAAAAAGTATA
TATATACGTATATATATATATATATATACACGTATATATATATACGT
ATATATATATACGTATATATATGCGTATATATATATACGTATATATA
TATACGTATATATATACGTATATATATATACGTATATATATACGTAT
ATATATACGTATATATATGAGTTATAAAAAGAGGTATAAATAACT
TTTGACGGATTTTATTTATTTCAAATTTGCAATGTTCTTTAAGTATC
TGGAGAATTGATGAAAATTAACACTCGTCAATTCTTACGCCTGGAAC

GACCTAGTTCTGATTGTATCCTCTACCTATCATCTATCTATTTATAT
CTATCTATCTATCTAGGGGGATATGTTTCTATGAGGCAAAAA**GTATA**
**TATA**TACGTATATATATATATATATATACACGTATATATATATAC**GT**
**ATATATAT**ATACGTATATATATGCGTATATATATATAC**GTATATATA**
TATAC**GTATATATAT**ACGTATATATATATAC**GTATATATAT**ACG**TAT**
**ATATATAC**GTATATATATGAGTTATAAAAAGAGGTATAAATAACT
TTTGACGGATTTTATTTATTTCAAATTTGCAATGTTCTTTAAGTATC
TGGAGAATTGATGAAAATTAACACTCGTCAATTCTTACGCCTGGAAC

# Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0018954 A
- US 5858659 A, Sapolsky
- US 6223127 B, Berno
- US 5166320 A
- US 4868116 A
- US 4980286 A
- WO 8907136 A
- WO 8902468 A
- WO 8905345 A
- WO 9207573 A
- US 5399346 A, Anderson
- WO 9505452 A, Baetge
- WO 60384122 A

**Non-patent literature cited in the description**

- **HORWITZ et al.** *J Clin Endocrinol Meta.,* February 2003, vol. 88 (2), 569-575
- **KARAPLIS et al.** *Genes Dev.,* 1994, vol. 8, 277-289
- **AMIZUKA et al.** *J. Cell Biol.,* 1994, vol. 126, 1611-1623
- **AMIZUKA et al.** *Developmental Biol.,* 1996, vol. 175, 166-176
- **STEWART et al.** *J. Bone Miner Res,* 2000, vol. 15, 1517
- **SAMBROOK et al.** Molecular Cloning - A Laboratory Manual. Cold Spring Harbour Laboratories, 1989
- **AUSUBEL et al.** Current Protocols in Molecular Biology. Wiley, 1994
- **GLICK et al.** Molecular Biotechnology - Principles and Applications of Recombinant DNA. ASM, 1994
- **ACSADI et al.** *Nature,* 1991, vol. 332, 815-818
- **WOLFF et al.** *Science,* 1990, vol. 247, 1465-1468
- **WU, G. ; WU, C. H.** *J. Biol. Chem.,* 1988, vol. 263, 14621
- **WILSON.** *J. Biol. Chem.,* 1992, vol. 267, 963-967
- **CURIEL.** *Proc. Natl. Acad. Sci. USA,* 1991, vol. 88, 8850
- **CRISTIANO et al.** *Proc. Natl. Acad. Sci. USA,* 1993, vol. 90, 2122-2126
- **MILLER, A. D.** *Blood,* 1990, vol. 76, 271
- **AUSUBEL, F. M. et al.** Current Protocols in Molecular Biology. Greene Publishing Associates, 1989
- **EGLITIS et al.** *Science,* 1985, vol. 230, 1395-1398
- **DANOS ; MULLIGAN.** *Proc. Natl. Acad. Sci. USA,* 1988, vol. 85, 6460-6464
- **WILSON et al.** *Proc. Natl. Acad. Sci. USA,* 1988, vol. 85, 3014-3018
- **ARMENTANO et al.** *Proc. Natl. Acad. Sci. USA,* 1990, vol. 87, 61416145
- **HUBER et al.** *Proc. Natl. Acad. Sci. USA,* 1991, vol. 88, 80398043
- **FERRY et al.** *Proc. Natl. Acad. Sci. USA,* 1991, vol. 88, 83778381
- **CHOWDHURY et al.** *Science,* 1991, vol. 254, 1802-1805
- **VAN BEUSECHEM et al.** *Proc. Natl. Acad. Sci. USA,* 1992, vol. 89, 7640-7644
- **KAY et al.** *Human Gene Therapy,* 1992, vol. 3, 641-647
- **DAI et al.** *Proc. Natl. Acad. Sci. USA,* 1992, vol. 89, 10892-10895
- **HWU et al.** *J. Immunol.,* 1993, vol. 150, 4104-4115
- **BERKNER et al.** *BioTechniques,* 1988, vol. 6, 616
- **ROSENFELD et al.** *Science,* 1991, vol. 252, 431-434
- **ROSENFELD et al.** *Cell,* 1992, vol. 68, 143-155
- **LEMARCHAND et al.** *Proc. Natl. Acad. Sci. USA,* 1992, vol. 89, 6482-6486
- **HERZ ; GERARD.** *Proc. Natl. Acad. Sci. USA,* 1993, vol. 90, 28122816
- **QUANTIN.** *Proc. Natl. Acad. Sci. USA,* 1992, vol. 89, 2581-2584
- **MUZYCZKA et al.** *Curr. Topics in Micro. and Immunol.,* 1992, vol. 158, 97-129
- **FLOTTE et al.** *Am. J. Respir. Cell. Mol. Biol.,* 1992, vol. 7, 349-356
- **SAMULSKI et al.** *J. Virol.,* 1989, vol. 63, 3822-3828
- **MCLAUGHLIN et al.** *J. Virol.,* 1989, vol. 62, 19631973

- **TRATSCHIN et al.** *Mol. Cell. Biol.,* 1985, vol. 5, 3251-3260
- **HERMONAT et al.** *Proc. Natl. Acad. Sci. USA,* 1984, vol. 81, 6466-6470
- **TRATSCHIN et al.** *Mol. Cell. Biol.,* 1985, vol. 4, 2072-2081
- **WONDISFORD et al.** *Mol. Endocrinol.,* 1988, vol. 2, 32-39
- **TRATSCHIN et al.** *J. Virol.,* 1984, vol. 51, 611619
- **FLOTTE et al.** *J. Biol. Chem.,* 1993, vol. 268, 3781-3790
- **CLAPP, D. W. et al.** *Blood,* 1991, vol. 78, 1132-1139
- **ANDERSON.** *Science,* 2000, vol. 288, 627-9
- **CAVAZZANA-CALVO et al.** *SCIENCE,* 2000, vol. 288, 669-72
- **HE et al.** *Endocrinology,* 2001, vol. 142 (5), 2070-7
- **YASUDA T. et al.** *J Biol Chem.,* 05 May 1989, vol. 264 (13), 7720-5
- **PAUSOVA Z. et al.** *Genomics,* 1993, vol. 17 (1), 243-4
- Techniques in Biochemistry and Molecular Biology--Hybridization with Nucleic Probes. **TIJSSEN.** Overview of principles of hybridization and the strategy of nucleic acid assays. Elsevier Science, Inc, 1993


SEQUENCE LISTING

<110> McGILL UNIVERSITY

<120> PTHrP-BASED PREDICTION AND DIAGNOSIS OF BONE DISEASE

<130> 9-16408-1PCT

<150> 60/384,122
<151> 2002-05-31

<160> 1

<170> Patent In Ver. 2.1

<210> 1
<211> 36
<212> DNA
<213> Artificial Sequence

<223> Description of Artificial Sequence: oligonucleotide

<400> 1
tatatacgta tatatatata cgtatatata tacgta          36